# EUROPÄISCHE PATENTANMELDUNG

(11) **EP 2 163 225 A1**
(43) Veröffentlichungstag der Anmeldung: **17.03.2010**
(21) Anmeldenummer: 08016045.0
(22) Anmeldetag: 11.09.2008
(51) Int. Cl.: A61F 2/38

(54) **Künstliches Gelenk mit einem polymeren Funktionskörper**

(71) Anmelder: Aequos Endoprothetik Gmbh, 82166 Gräfelfing (DE)
(72) Erfinder: Nägerl, Hans, 37130 Gleichen (DE)
(74) Vertreter: Scheffler, Jörg

(57) **Zusammenfassung**

Die Erfindung betrifft ein künstliches Gelenk als Endoprothese für ein menschliches Kniegelenk. Das künstliche Gelenk dient als Endoprothese für ein menschliches Kniegelenk, wobei ein Funktionskörper (3) mit einer eine Artikulationsfläche bildenden Funktionsfläche ausgestattet ist. Der Funktionskörper (3) hat eine die Funktionsfläche umfangsseitig einschließende, ringförmig geschlossene Aufnahme (2) mit einem Rand (6), welcher mit der Funktionsfläche konturbündig abschließt, sodass also die Höhe (H, h) der Aufnahme (2) bis zu dem Rand (6) der jeweiligen Materialstärke des Funktionskörpers (3) unabhängig von einer möglichen Erhöhungen (4) oder Vertiefung (5) des Funktionskörpers (3) entspricht. Hierdurch wird insbesondere die mittlere Werkstoffbeanspruchung verringert, indem das seitliche Fließen des polymeren Werkstoffs dadurch verhindert wird, dass die Aufnahme (2) einer Formänderung des Funktionskörpers (3) einen Widerstand entgegensetzt.

## Beschreibung

Die Erfindung betrifft ein künstliches Gelenk als Endoprothese für ein menschliches Kniegelenk mit einem einem ersten Gelenkteil zugeordneten polymeren Funktionskörper, welcher mit einer eine Artikulationsfläche für das zweite Gelenkteil aufweisenden Funktionsfläche ausgestattet ist. Weiterhin betrifft die Erfindung einen derartigen Funktionskörper aus einem Polymer zur Verwendung bei einem künstlichen Gelenk.

Ein solches Gelenk sowie ein derartiger, oftmals als Inlay bezeichneter Funktionskörper aus einem hochmolekularen und teilkristallisierten Polyethylen (UHMWPE) werden bereits in großer Stückzahl in Hüft- und Kniegelenken als Implantate eingesetzt.

Beispielsweise beschreibt die DE 197 05 733 A1 einen Tibiateil einer Kniegelenkendoprothese mit einer Tibia-Plattform und einem an der distalen Seite derselben angeordneten Verankerungselement. Die Tibia-Plattform dient der Aufnahme einer als Polyethyleninlay ausgeführten Funktionsfläche, wobei die Tibia-Plattform eine Bodenfläche sowie einen geschlossenen Rand aufweist, welcher gegenüber der Bodenfläche erhaben ist.

Bei der WO 02/078656 A2 hat die Aufnahme einen den Funktionskörper umfangsseitig einschließenden Rand und eine mit dem Funktionskörper zusammenwirkende Hinterschneidung.

Weiterhin offenbart die DE 89 09 036 U1 ein Tibiateil einer Kniegelenk-Endoprothese mit einem Tibiaplateau und einem von der tibialen Stützfläche des Tibiaplateaus abwärts gerichteten Schaft zur Verankerung im Markraum des natürlichen Tibiaknochens. Das Tibiaplateau besteht aus einer am Schaft befestigten unteren Platte, auf der eine Auflage aus Kunststoffmaterial befestigt ist.

Die DE 43 22 619 C1 betrifft ein Tibiateil einer Kniegelenk-Endoprothese mit einem metallischen Tibiaplateau, einem Kunststoffeinsatz auf dem Tibiaplateau und einem in den Markraum eintreibbaren zentralen Stiel unter dem Tibiaplateau. Der Kunststoffeinsatz wird durch einen proximal von dem Tibiaplateau abzweigenden Randflansch gegen seitliches Verrutschen gesichert.

Durch den Stand der Technik sind ferner auch bereits eine Vielzahl von Endoprothesen für ein menschliches Hüftgelenk bekannt. Beispielsweise bezieht sich die DE 196 54 409 C5 auf eine endoprothetische Hüftgelenkpfanne, die eine Außenschale und eine Innenschale aufweist, wobei die Innenschale auf wenigstens einem Teil ihrer Außenseite über einen Klemmsitz in wenigstens einem Teilbereich der Innenseite der Außenschale festlegbar ist. Die Innenschale kann aus einem geeigneten Kunststoff, beispielsweise UHMWPE, bestehen. Eine solche Innenschale aus Kunststoff wird je nach den Eigenschaften des verwendeten Kunststoffs mit einer Anzahl nicht dargestellter Vorsprünge oder einer umlaufenden Rippe zum Eingriff in eine Nut auf der Innenseite der Außenschale versehen.

Die DE 44 02 675 A1 betrifft eine Hüftgelenkpfanne zum Einsetzen in Knochengewebe mit einer äußeren Metallschale und einer inneren Gleitschale, wobei die Auflagefläche der Gleitschale in der Metallschale konisch ausgebildet ist. Die Gleitschale überragt einen oberen Rand der Metallschale geringfügig.

Der polymere Werkstoff begrenzt im Normalfall bei derartigen Endoprothesen die Lebensdauer des Funktionskörpers aufgrund seines visko-elastischen und -plastischen Verformverhaltens mit relativ kleinem, ideal-elastischen Bereich von nur einigen MPa.

Der polymere Funktionskörper zeigt bereits bei kleinen zyklischen Belastungen eine zyklische Verformung, die der Belastung nachfolgt, sodass sich für die Belastungs-Verformungs-Charakteristik eine Hysterese ergibt. Das Flächenintegral der Hysterese stellt die Verlustarbeit dar, die der Wärmeentwicklung entspricht.

In Totalendoprothesen unterliegt das UHMWPE-Inlay nur einer reinen Druckbelastung. Schon die statische Druck-Verformungs-Charakteristik ist komplex: Nach anfangs linearem Anstieg nimmt mit zunehmender Verformung die Druckbelastung überproportional zu, bis sich schließlich die Krümmung der Charakteristik ändert und bei weiter zunehmender Verformung die Druckbelastung asymptotisch auf einen konstanten Wert zugeht, was anzeigt, dass sich jetzt ein Kaltflusszustand eingestellt hat. Bei zyklischen Druckverformungen, wie sie in Totalendoprothesen - beispielsweise Knieendoprothesen - auftreten, entsteht auch wieder eine Hysterese, die Wärmeentwicklung bedeutet. Wärmeentwicklung begünstigt aber wieder den Kaltfluss und damit die Zerstörung des Inlays. Das kann schon nach verhältnismäßig wenigen Lastwechseln auftreten. Der als Folge der Druckbelastung auftretende Fließweg wird bei makroskopischem Ausmaß als Kaltfluss bezeichnet.

Der Erfindung liegt daher die Aufgabe zu Grunde, das Ausfallrisiko eines solchen Formkörpers wesentlich zu verringern. Diese Aufgabe wird erfindungsgemäß mit einem künstlichen Gelenk gemäß den Merkmalen des Anspruchs 1 gelöst. Die weitere Ausgestaltung der Erfindung ist den Unteransprüchen zu entnehmen.

Erfindungsgemäß ist also ein künstliches Gelenk vorgesehen, bei dem der Funktionskörper mit seiner der Funktionsfläche abgewandten Auflagefläche auf einer Bodenfläche einer Aufnahme aufliegt, wobei die Aufnahme eine den Funktionskörper umfangsseitig einschließenden, ringförmig geschlossenen Rand aufweist, welcher gegenüber der Bodenfläche vorspringend sowie mit der Funktionsfläche zumindest annähernd konturbündig abschließend ausgeführt ist, und die Aufnahme den Funktionskörper kraftschlüssig und formschlüssig einschließt. Hierdurch wird erfindungsgemäß mit einem vergleichsweise geringen Aufwand erreicht, dass die effektive Stauchung bzw. Dehnung des Funktionskörpers wesentlich herabgesetzt wird, was zu einer signifikanten Lebensdauererhöhung des polymeren Funktionskörpers, welcher zumindest als wesentlichen Materialbestandteil ein hochmolekulares und teilkristallisiertes PE, insbesondere UHMWPE enthält, und damit des künstlichen Gelenks führt. Insbesondere wird so die Werkstoffbeanspruchung verringert, indem das nach dem Stand der Technik unvermeidliche Fließen durch inneres, insbesondere seitliches Abscheren herabgesetzt wird. Die Höhendifferenz zwischen dem Rand und der Funktionsfläche ist dabei so gering wie möglich, zumindest aber kleiner als 1 mm bemessen, wobei die Aufnahme in der Praxis bis zur vollen Höhe der Funktionsfläche reicht. Die Aufnahme setzt also im Gebrauch einer Formänderung des Funktionskörpers quer zur Belastungsrichtung ein Widerlager entgegen.

Die Aufnahme ist dabei derart ausgeführt, dass mittels dieser in den Funktionskörper ein Druck derart eingebracht wird, dass dieser die durch den Belastungszustand eingebrachten Belastungen ganz oder teilweise kompensiert, somit also zu einem Spannungsausgleich führt. Dabei schließt die Aufnahme den Funktionskörper form- und kraftschlüssig ein, wobei die Aufnahme eine mit dem Funktionskörper zusammenwirkende Hinterschneidung aufweisen kann. Mittels der Aufnahme kann eine Vorspannkraft auf den Funktionskörper übertragen werden, die auf eine geometrische Mitte der Funktionsfläche oder deren Flächenschwerpunkt gerichtet ist und die entsprechend dem visko-elastischen Verformungsverhalten des Funktionskörpers partiell differieren kann. Das beim Stand der Technik in der Praxis zu einer Schädigung der Funktionsfläche führende Fließen kann aufgrund der als seitlicher Gegendruck wirkenden Vorspannkraft zumindest wesentlich vermindert bzw. verzögert oder vollständig vermieden werden. Die mittels der Aufnahme auf den Funktionskörper übertragbare Vorspannkraft ist dabei entsprechend dem elastischen oder plastischen Verformungsverhalten des Funktionskörpers und/oder der Beanspruchung aufgrund der Gelenkbewegung bestimmt.

Hierzu ist der Rand vorzugsweise als eine insbesondere rechtwinklige Abwinkelung gegenüber der Bodenfläche ausgeführt und fasst daher den Funktionskörper umlaufend und konturbündig ein. Selbstverständlich können der Rand und die Bodenfläche als separate, miteinander dauerhaft verbundene Bauteile ausgeführt oder aber einteilig miteinander verbunden sein.

Der Einsatz eines derartigen Funktionskörpers als ein Tibia-Gelenkersatz ist dabei nicht auf Endoprothesen für ein menschliches Kniegelenk beschränkt. Vielmehr lässt sich dieses Prinzip in vorteilhafter Weise auch auf weitere Endoprothesen, beispielsweise für ein menschliches Fingergelenk, einsetzen.

Dabei ist es besonders zweckmäßig, wenn die Aufnahme den Funktionskörper form- und kraftschlüssig einschließt, wobei die Aufnahme eine mit dem Funktionskörper zusammenwirkende Hinterschneidung aufweist. Hierdurch wird mittels der Aufnahme eine Vorspannkraft auf den Funktionskörper übertragen, die auf eine geometrische Mitte der Funktionsfläche oder deren Flächenschwerpunkt gerichtet ist und die entsprechend des elastischen Verformungsverhaltens der Aufnahme partiell differieren kann. Das beim Stand der Technik in der Praxis zu einer Schädigung der Funktionsfläche führende Fließen kann aufgrund der als seitlicher Gegendruck wirkenden Vorspannkraft zumindest wesentlich vermindert bzw. verzögert oder vollständig vermieden werden. Die mittels der Aufnahme auf den Funktionskörper übertragbare Vorspannkraft ist dabei entsprechend des elastischen oder plastischen Verformungsverhaltens des Funktionskörpers und/oder der Beanspruchung aufgrund der Gelenkbewegung bestimmt.

Die Funktionsfläche weicht in der Praxis von einer Kreisform ab. Somit erweist es sich als besonders Erfolg versprechend, wenn mittels der Aufnahme eine der begrenzenden Kontur der Funktionsfläche entsprechende, partiell abweichende Vorspannkraft realisierbar ist. Hierdurch wird ein einheitlicher Spannungszustand in der Funktionsfläche ohne unerwünschte Spannungsspitzen sichergestellt.

Zu diesem Zweck weist die Aufnahme eine der gewünschten Vorspannkraft entsprechende Materialstärke, Querschnittsform, Querschnittsfläche und/oder Breite auf.

Die Aufnahme ist umfangsseitig bevorzugt durch eine geschlossene Fläche gebildet. Dabei sind jedoch auch solche praxisgerechten Abwandlungen von dem Erfindungsgedanken umfasst, bei denen der Rand am Umfang des Funktionskörpers abschnittsweise Ausnehmungen aufweist, wobei die Ausnehmungen derart bemessen sind, dass ein Fließen in die Ausnehmungen hinein oder hindurch weitgehend ausgeschlossen ist.

Eine besonders praxisgerechte Ausgestaltung der vorliegenden Erfindung wird auch dann erreicht, wenn die Aufnahme eine den Rand aufweisende, den Funktionskörper umfangsseitig einschließende Wandfläche sowie eine mit der Wandfläche verbundene Bodenfläche aufweist und so beispielsweise als ein becher- oder napfartig geformter, einseitig geschlossener Hohlkörper ausgeführt ist, welcher den Funktionskörper unter Auslassung der Funktionsfläche vollständig einschließt.

Bevorzugt ist die Aufnahme formstabil ausgeführt und besteht zu diesem Zweck aus einem Metall oder einer Keramik, um so eine steife Einfassung zu erzeugen. Zumindest teilelastische Eigenschaften können vorzugsweise zur Realisierung der Vorspannkraft genutzt werden. Dadurch ergeben sich für die Aufnahme eine gute Wärmeleitfähigkeit, um so die durch die Formänderung entstehende Wärme schnell abführen zu können und ein unerwünschtes Erweichen des polymeren Funktionskörpers zu vermeiden. Eine Temperaturerhöhung mit der Folge einer dauerhaften Schädigung des Werkstoffes wird dadurch wirksam vermieden. Durch die bevorzugte Einfassung der Aufnahme aus Metall wird die Wärmeableitung optimiert.

Die Erfindung lässt verschiedene Ausführungsformen zu. Zur weiteren Verdeutlichung ihres Grundprinzips ist eine davon in der Zeichnung dargestellt und wird nachfolgend beschrieben. Diese zeigt in
- Fig. 1: eine Draufsicht auf einen für ein künstliches Gelenk bestimmten polymeren Funktionskörper;
- Fig. 2A: eine Seitenansicht aus der Richtung A des in Figur 1 gezeigten Funktionskörpers:
- Fig. 2B: eine Seitenansicht aus der Richtung B des in Figur 1 gezeigten Funktionskörpers;
- Fig. 3: beispielhaft in einer geschnittenen Seitenansicht einen weiteren Funktionskörper.

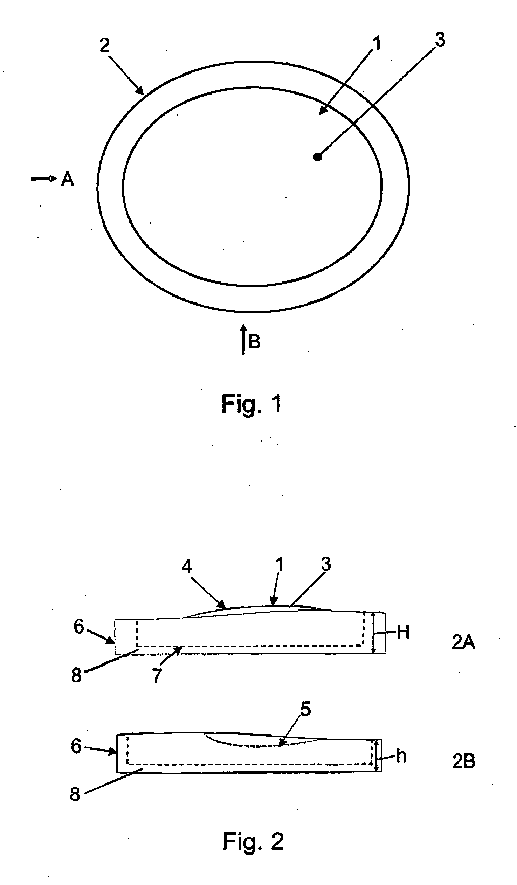

Das erfindungsgemäße künstliche Gelenk wird nachstehend anhand der Figuren 1, 2A und 2B näher dargestellt, welche jeweils einen zum Einsatz bei dem künstlichen Gelenk bestimmten polymeren Funktionskörper 3 als inlay zeigen. Das künstliche Gelenk dient als Endoprothese für ein menschliches Kniegelenk, wobei der Funktionskörper 3 mit einer Funktionsfläche 1 ausgestattet ist, die zugleich eine Artikulationsfläche für ein hierzu relativ bewegliches, nicht gezeigtes Gelenkteil bildet. Der Funktionskörper 3 liegt mit seiner der Funktionsfläche 1 abgewandten Auflagefläche 7 auf einer Bodenfläche 8 einer Aufnahme 2 auf, die mit einem die Funktionsfläche 1 umfangsseitig einschließenden, ringförmig geschlossenen Rand 6 ausgestattet ist. Der Rand 6 schließt mit der Funktionsfläche konturbündig ab, sodass also die Höhe H bzw. h der Aufnahme 2 bis zu dem Rand 6 der jeweiligen Materialstärke des Funktionskörpers 3 unabhängig von einer möglichen Erhöhungen 4 oder Vertiefung 5 des Funktionskörpers 3 entspricht. Hierdurch wird insbesondere die Dehnung bzw. Stauchung und dadurch die mittlere Belastungsspannung herabgesetzt, indem das seitliche Fließen des polymeren Werkstoffs dadurch verhindert wird, dass die Aufnahme 2 einer Formänderung des Funktionskörpers 3 einen Widerstand entgegensetzt.

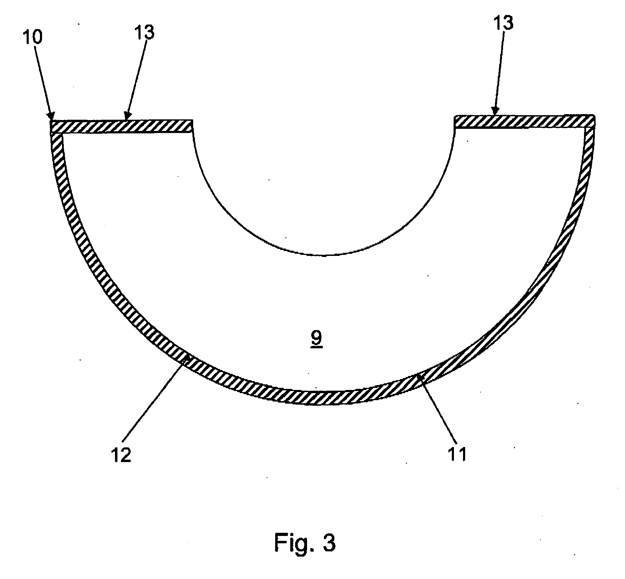

In Figur 3 ist lediglich beispielhaft im Querschnitt ein weiterer Funktionskörper 9 zu erkennen, welcher in einer konkaven Aufnahme 10 fixiert ist. Eine entsprechend geformte Auflagefläche 11 auf einer Bodenfläche 12 der Aufnahme 10 ist an ihrer Oberseite mit einem ringförmig geschlossenen Rand 13 versehen, welcher gegenüber der Bodenfläche 12 nach innen vorspringend ausgeführt ist. Der Rand 13 schließt mit dem Funktionskörper 9 zumindest annähernd konturbündig ab, sodass eine Verformung des polymeren Funktionskörpers 9 auch unter hoher Belastung nahezu ausgeschlossen ist. Zugleich kann durch die kraftschlüssige und formschlüssige Aufnahme 10 eine Vorspannkraft auf den Funktionskörper 9 übertragen werden, um so die Belastbarkeit wesentlich zu erhöhen.

## Patentansprüche

1. Künstliches Gelenk als Endoprothese für ein menschliches Kniegelenk mit einem einem ersten Gelenkteil zugeordneten polymeren Funktionskörper (3, 9), welcher mit einer eine Artikulationsfläche für ein zweites Gelenkteil aufweisenden, in einer Aufnahme (2, 10) gehaltenen Funktionsfläche (1) ausgestattet ist, wobei der Funktionskörper (3, 9) mit seiner der Funktionsfläche (1) abgewandten Auflagefläche (7, 11) auf einer Bodenfläche (8, 12) der Aufnahme (2, 10) aufliegt und die Aufnahme (2, 10) einen den Funktionskörper (3, 9) umfangsseitig einschließenden, umlaufend geschlossenen Rand (6, 13) aufweist, welcher gegenüber der Bodenfläche (8, 12) vorspringend sowie mit der Funktionsfläche (1) zumindest annähernd konturbündig abschließend ausgeführt ist, und die Aufnahme (2, 10) den Funktionskörper (3, 9) kraftschlüssig und formschlüssig einschließt.

2. Künstliches Gelenk nach Anspruch 1, **dadurch gekennzeichnet, dass** mittels der Aufnahme in den Funktionskörper (3, 9) ein Druck, insbesondere eine Vorspannung, derart eingebracht wird, dass dieser die durch den Belastungszustand eingebrachten Belastungen ganz oder teilweise kompensiert.

3. Künstliches Gelenk nach den Ansprüchen 1 oder 2, **dadurch gekennzeichnet, dass** die Aufnahme (2, 12) eine mit dem Funktionskörper (3, 9) zusammenwirkende Hinterschneidung aufweist.

4. Künstliches Gelenk nach zumindest einem der vorhergehenden Ansprüche, **dadurch gekennzeichnet, dass** der Rand (6, 13) als eine insbesondere rechtwinklige Abwinkelung gegenüber der Bodenfläche (8, 12) ausgeführt ist.

5. Künstliches Gelenk nach zumindest einem der vorhergehenden Ansprüche, **dadurch gekennzeichnet, dass** der Rand (6, 13) und die Bodenfläche (8, 12) einteilig miteinander verbunden sind.

6. Künstliches Gelenk nach zumindest einem der vorhergehenden Ansprüche, **dadurch gekennzeichnet, dass** der Rand (6, 13) außerhalb einer Artikulationsfläche für das zweite Gelenkteil angeordnet ist.

7. Künstliches Gelenk nach zumindest einem der vorhergehenden Ansprüche, **dadurch gekennzeichnet, dass** der Funktionskörper (3, 9) als ein Tibia-Gelenkersatz ausgeführt ist.

8. Künstliches Gelenk nach zumindest einem der vorhergehenden Ansprüche, **dadurch gekennzeichnet, dass** eine mittels der Aufnahme (2, 12) auf den Funktionskörper (3, 9) übertragbare Vorspannkraft entsprechend dem elastischen und/oder plastischen Verformungsverhalten des Funktionskörpers (3, 9) und/oder der Beanspruchung aufgrund der Gelenkbewegung bestimmt ist.

9. Künstliches Gelenk nach zumindest einem der vorhergehenden Ansprüche, **dadurch gekennzeichnet, dass** mittels der Aufnahme (2, 12) eine der begrenzenden Kontur der Funktionsfläche (1) entsprechende, partiell abweichende Vorspannkraft realisierbar ist.

10. Künstliches Gelenk nach zumindest einem der vorhergehenden Ansprüche, **dadurch gekennzeichnet, dass** die Aufnahme (2, 12) eine der gewünschten Vorspannkraft entsprechende Materialstärke, Querschnittsform, Querschnittsfläche und/oder Breite aufweist.

11. Künstliches Gelenk nach zumindest einem der vorhergehenden Ansprüche, **dadurch gekennzeichnet, dass** der Rand (6, 13) am Umfang des Funktionskörpers (3, 9) abschnittsweise Ausnehmungen aufweist.

12. Künstliches Gelenk nach zumindest einem der vorhergehenden Ansprüche, **dadurch gekennzeichnet, dass** die Aufnahme (2, 12) eine den Rand (6, 13) aufweisende, den Funktionskörper (3, 9) umfangsseitig einschließende Wandfläche sowie eine mit der Wandfläche verbundene Bodenfläche (8, 12) aufweist.

13. Künstliches Gelenk nach zumindest einem der vorhergehenden Ansprüche, **dadurch gekennzeichnet, dass** die Aufnahme (2, 12) formstabil ausgeführt ist.

14. Künstliches Gelenk nach zumindest einem der vorhergehenden Ansprüche, **dadurch gekennzeichnet, dass** die Aufnahme (2, 12) aus einem Metall und/oder einer Keramik besteht.

15. Polymerer Funktionskörper (3, 9) mit einer Funktionsfläche (1) zur Verwendung bei einem künstlichen Gelenk nach zumindest einem der vorhergehenden Ansprüche, **gekennzeichnet durch** eine die Funktionsfläche (1) umfangsseitig einschließende, geschlossene Aufnahme (2) mit einem Rand (6, 13), welcher mit der Funktionsfläche (1) zumindest annähernd konturbündig abschließend ausgeführt ist.
